# EUROPÄISCHE PATENTANMELDUNG

(11) **EP 4 725 932 A1**
(43) Veröffentlichungstag der Anmeldung: **15.04.2026**
(21) Anmeldenummer: 24205324.7
(22) Anmeldetag: 08.10.2024
(51) Int. Cl.: C07C 29/151, C07C 31/04

(54) **VERFAHREN UND ANLAGE ZUR SYNTHESE VON EMETHANOL MIT HOHER WASSERSTOFFAUSNUTZUNG**

(71) Anmelder: GasConTec GmbH, 61348 Bad Homburg v. d. Höhe (DE)
(72) Erfinder: LAUER, Wolfgang, 34613 Schwalmstadt (DE); RAPPOLD, Dorit, 60437 Frankfurt (DE); WAGNER, Ulrich, 06406 Bernburg (DE); GNAGNETTI, Andrea, 20851 Lissone (MB) (IT)
(74) Vertreter: Patentanwälte Bauer Vorberg Kayser

(57) **Zusammenfassung**

Die Erfindung betrifft ein Verfahren zur Synthese von eMethanol (31), wobei ein überwiegend aus Kohlenstoffdioxid bestehender CO2-Strom (1) und ein überwiegend aus Wasserstoff bestehender H-Strom (2) einer Methanol-Reaktoranordnung (7) zur Umwandlung in eMethanol (31) zugeführt werden, wobei ein Restgasstrom (19) mit unreagiertem Wasserstoff und unreagiertem Kohlenstoffdioxid aus der Methanol-Reaktoranordnung (7) hervorgeht, wobei die Methanol-Reaktoranordnung (7) einen ersten adiabatischen Pre-Reaktor (12) und einen nachgeschalteten zweiten isothermen Reaktor (13) aufweist, wobei aus dem ersten Pre-Reaktor (12) ein erster Rohmethanol-Teilstrom (15) gewonnen wird und ein Pre-Restgasstrom (16), wobei der Pre-Restgasstrom (16) dem zweiten Reaktor (13) zur Gewinnung von Dampf (29), eines zweiten Rohmethanol-Teilstroms (18) und des Restgasstromes (19) zugeführt wird und wobei ein Teil des Restgasstromes (19a) einer Wasserstoffrückgewinnungsanordnung (22) zugeführt wird zum Gewinnen eines Wasserstoff-Rückführungsstroms (3).

## Beschreibung

Die Erfindung betrifft ein Verfahren zur Synthese von eMethanol gemäß dem Oberbegriff von Anspruch 1 sowie eine Anlage zur Synthese von eMethanol gemäß dem Oberbegriff des Anspruchs 15.

Die Herstellung von Methanol erfolgt durch die Reaktion von Wasserstoff und Kohlenstoffoxiden, welche einem Methanol-Reaktor als Gasströme zugeführt werden. Die benötigten Edukte Wasserstoff und Kohlenstoffoxid können aus unterschiedlichen Quellen gewonnen werden. Üblicherweise werden die Edukte dabei in bekannter Art und Weise aus einem kohlenstoffhaltigen Energieträgerstrom, wie z. B. Erdgas gewonnen. Bei der Herstellung von sogenanntem eMethanol stammen die Edukte aus nicht-fossilen, nachhaltigen Quellen.

Für die Herstellung von eMethanol kann ein Kohlenstoffdioxid-reicher Gasstrom verwendet werden, der keinen Wasserstoff enthält. Ein solcher Gasstrom kann etwa aus einem Rauchgas gewonnen werden, also aus einem Abgas einer Verbrennung von nicht-fossilen Rohstoffen, z.B. Holz. Ein Kohlenstoffdioxid-reicher Gasstrom kann alternativ aus einer Anlage zum Herstellen von Bioethanol oder Biomethan (z.B. mittels anaerober Verdauung) gewonnen werden.

Der ebenfalls benötigte Wasserstoff muss dann aus einer anderen Quelle zugeführt werden. Der Wasserstoff kann unter anderem durch Elektrolyse gewonnen werden, in welcher Wasser durch den Einsatz von Energie in die Bestandteile Wasserstoff und Sauerstoff getrennt wird. Gerade die separate Gewinnung von Wasserstoff ist sehr energieintensiv und der so gewonnene Rohstoff Wasserstoff besonders teuer.

Hinzu kommt, dass die Umsetzung von CO2 und Wasserstoff zu eMethanol in einer Methanol-Reaktoranordnung unvollständig erfolgt. In der Regel können Umsetzungsraten zwischen 93% und 97% erzielt werden. Das heißt, dass ca. 3% bis 7% des eingesetzten Wasserstoffs nicht zur Herstellung des eMethanols beitragen. Weiterhin nimmt der Verbrauch an Wasserstoff noch zu, wenn die Destillation von eMethanol - unter Vermeidung von CO2-Ausstoß - durch die Verbrennung von weiterem Wasserstoff erfolgt.

Der Einsatz von Wasserstoff macht den Prozess besonders teuer, da die Elektrolyse von Wasserstoff sehr energieintensiv ist. So werden für die Herstellung von 1 Tonne Wasserstoff 50 MWh benötigt. Bei der Herstellung von 400 Tonnen eMethanol bedeutet ein Verlust von 5% Wasserstoff einen zusätzlichen Energieaufwand von 12MWh.

Die WO 2020/229261 A1, von welcher die vorliegende Erfindung als nächstkommend ausgeht, beschreibt ein Verfahren zur Synthese von Methanol mit hoher Wasserstoffausnutzung. Allerdings wird hier vorgeschlagen zwei isotherme Reaktoren hintereinanderzuschalten, um eine möglichst große Umsetzung von Wasserstoff zu ermöglichen. Dies führt aber zu einer insgesamt sehr großen Reaktorfläche, welche das Verfahren wiederrum besonders teuer machen.

Die zugrundeliegende Aufgabe der Erfindung ist es, ein Verfahren und eine Anlage zur Synthese von eMethanol zur Verfügung zu stellen, welche besonders kostengünstig und energieeffizient sind. Insbesondere soll die Ausnutzung des gewonnen Wasserstoffes besonders hoch sein.

Die Lösung der Aufgabe erfolgt erfindungsgemäß mit den Merkmalen der unabhängigen Ansprüche. Weitere Vorteile und praktische Ausführungsformen sind in Verbindung mit den abhängigen Ansprüchen beschrieben.

Bei dem erfindungsgemäßen Verfahren zur Synthese von eMethanol werden ein überwiegend aus Kohlenstoffdioxid bestehender CO2-Strom und ein überwiegend aus Wasserstoff bestehender H-Strom einer Methanol-Reaktoranordnung zur Umwandlung in eMethanol zugeführt. Ein überwiegend aus Kohlenstoffdioxid bestehender CO2-Strom bedeutet, dass der molare Anteil von Kohlenstoffdioxid des CO2-Stroms mindestens 80% beträgt. Vorzugsweise beträgt der molare Anteil von Kohlenstoffdioxid des CO2-Stroms mindestens 95 % und insbesondere mindestens 99 %. Dass der H-Strom überwiegend aus Wasserstoff besteht, bedeutet entsprechend, dass der molare Anteil von Wasserstoff des H-Stroms mindestens 90% beträgt. Vorzugsweise beträgt der molare Anteil von Wasserstoff des H-Stroms mindestens 95 % und insbesondere mindestens 99 %. Insbesondere erfolgt die Gewinnung des CO2-Stroms über eine Rauchgaswäsche eines Rauchgase oder Abgases einer Anlage zur Herstellung von Bioethanol oder Biomethan. Der H-Strom wird insbesondere mittels Elektrolyse gewonnen.

Die Umsetzung zu eMethanol erfolgt dann in der Methanol-Reaktoranordnung. Die Umwandlung der zugeführten Edukte in eMethanol ist regelmäßig nicht vollständig, sodass ein Restgasstrom mit unreagiertem Wasserstoff und unreagiertem Kohlenstoffdioxid aus der Methanol-Reaktoranordnung hervorgeht. Der Restgasstrom kann neben dem unreagierten Wasserstoff auch unreagiertes Kohlenstoffdioxid sowie in der Methanol-Reaktoranordnung insbesondere durch die umgekehrte Wassergas-Shift-Reaktion entstandenes Kohlenstoffmonoxid aufweisen. Ebenso kann der Restgasstrom inerte Bestandteile wie Stickstoff, Methan und Edelgase sowie Nebenprodukte wie Dimethylether aufweisen. Die inerten Bestandteile werden vor allem mit den Ausgangsprodukten H-Strom oder CO2-Strom eingebracht. Neben dem Restgasstrom wird auch ein Rohmethanolstrom aus der Methanol-Reaktoranordnung gewonnen.

Erfindungsgemäß weist die Methanol-Reaktoranordnung einen ersten adiabatischen Pre-Reaktor und einen nachgeschalteten zweiten isothermen Reaktor auf. Aus dem ersten Pre-Reaktor wird ein erster Rohmethanol-Teilstrom gewonnen und ein Pre-Restgasstrom, wobei der Pre-Restgasstrom dem zweiten Reaktor zur Gewinnung von Dampf, eines zweiten Rohmethanol-Teilstroms und des Restgasstromes zugeführt wird, insbesondere direkt zugeführt. Mit anderen Worten sind ein erster adiabatischer Pre-Reaktor und ein zweiter isothermer Reaktor in Serie geschaltet.

Bei einem adiabatischen Reaktor erfolgt keine Temperaturführung der Reaktion, insbesondere wird der adiabatische Reaktor nicht aktiv gekühlt. Der isotherme Reaktor ist insbesondere wassergekühlt auf vorzugsweise 200°C bis 300°C, insbesondere 250°C. Ein isothermer Reaktor zum Umsetzung von Synthesegas in eMethanol ist üblicherweise so aufgebaut, dass ein Bündel an Rohren gefüllt mit Katalysator von Kühlmedium umgeben ist. Solche isothermen Reaktoren sind besonders teuer und je größer das Volumen des isothermen Reaktors ist, umso höher sind die Kosten. Durch den davor angeordneten ersten Pre-Reaktor weist der zweite isotherme Reaktor ein vergleichsweise kleines Volumen auf.

Der Wirkungsgrad der Synthese kann insofern erhöht werden, als im ersten Methanol-Reaktor schon ein Teil des Synthesegases bestehend aus dem CO2-Strom und dem H-Strom in eMethanol umgesetzt wird. Vorzugsweise werden mindestens 10% und bevorzugt mindestens 20% des CO2 aus dem Synthesegas in eMethanol umgesetzt. Der zweite Reaktor kann dementsprechend kleiner ausgelegt werden, was zu einer deutlichen Kostensenkung führt.

Der erste Pre-Reaktor und der zweite Reaktor werden insbesondere auf einem im Wesentlichen gleichen Druckniveau betrieben, insbesondere bei 75 bar bis 100 bar, insbesondere bei ca. 80 bar.

Weiter erfindungsgemäß wird ein Teil des Restgasstromes einer Wasserstoffrückgewinnungsanordnung zugeführt zum Gewinnen eines Wasserstoff-Rückführungsstroms. Der Wasserstoff-Rückführungsstrom wird insbesondere der Methanol-Reaktoranordnung wieder zugeführt. Der Wasserstoff-Rückführungsstrom besteht überwiegend aus Wasserstoff. Das bedeutet, dass der molare Anteil von Wasserstoff im Wasserstoff-Rückführungsstrom mindestens 90 % beträgt. Vorzugsweise beträgt der molare Anteil von Wasserstoff im Wasserstoff-Rückführungsstrom mindestens 99 % und insbesondere mindestens 99,5 %. Bevorzugt ist ebenso, dass der molare Wasserstoffanteil in dem Wasserstoff-Rückführungsstrom höher als in dem Restgasstrom ist.

Durch die Gewinnung eines Wasserstoff-Rückführungsstromes mit einem sehr hohen Wasserstoffanteil aus dem Restgasstrom, wird der Anteil an inerten Bestandteilen in der Methanol-Reaktoranordnung reduziert.

Insgesamt ist das vorschlagsgemäße Verfahren auch dann gut geeignet, wenn der Anteil an inerten Bestandteilen im CO2-Strom hoch ist. Der Anteil an inerten Bestandteilen im CO2-Strom kann mindestens 1% oder maximal 15% betragen.

Ein Teil des Restgasstromes mit dem unreagierten Wasserstoff und dem unreagierten Kohlenstoffdioxid wird insbesondere als Recyclestrom der Methanol-Reaktoranordnung wieder zugeführt. Der Recyclestrom wird insbesondere Stromaufwärts der Wasserstoffrückgewinnungsanordnung von dem Restgasstrom abgezweigt. Insbesondere wird der Druck des Recyclestroms vor Rückführung zur Methanol-Reaktoranordnung durch eine Recycle-Kompressoranordnung erhöht.

Gemäß einer weiteren praktischen Ausführungsform des Verfahrens wird der Recyclestrom der Methanol-Reaktoranordnung stromabwärts des ersten Pre-Reaktors und stromaufwärts des zweiten Reaktors zugeführt. Insbesondere wird der Recyclestrom zusammen mit dem Pre-Restgasstrom aus dem ersten Pre-Reaktor dem zweiten Reaktor zugeführt. Durch die Rückführung des Recyclestroms in den zweiten Reaktor und nicht in den ersten Reaktor, kann der erste Reaktor weiterhin ein kleines Volumen aufweisen und mit nur einem geringen Volumen an Katalysator betrieben werden. Der erste Reaktor ist dadurch vergleichsweise kostengünstig.

Durch die - im Folgenden noch detaillierter beschriebene - Wasserstoffrückgewinnungsanordnung kann der Volumenstrom des Recyclestroms im Vergleich zu den bekannten Verfahren besonders klein gewählt werden, da der im Restgasstrom verbleibende Wasserstoff im Folgenden in der Wasserstoffrückgewinnungsanordnung bis auf einen kleinen Teil rückgewonnen werden kann. Der Volumenstrom des Recyclestroms, der von der Recycle-Kompressoranordnung druckerhöht werden muss, ist so besonders klein und der Energieverbrauch besonders niedrig.

Der Recyclefaktor des zweiten Reaktors kann maximal 3,5 betragen. Die effiziente Gewinnung eines Wasserstoff-Rückführungsstroms aus dem Restgasstrom mit der nachfolgenden Wasserstoffrückgewinnungsanordnung ermöglicht es, den Recyclefaktor gering zu halten und somit auch den isothermen zweiten Reaktor wie auch die Recycle-Kompressoranordnung vergleichsweise klein auszulegen.

In einer weiteren praktischen Ausführungsform wird der aus dem ersten Pre-Reaktor und einer nachfolgenden Methanol-Trennanordnung gewonnene Pre-Restgasstrom insbesondere direkt dem zweiten Reaktor zugeführt. Direkt bedeutet hier, dass der aus der Methanol-Trennanordnung gewonnene Pre-Restgasstrom insbesondere mit im Wesentlichen gleichem Druck und/oder mit im Wesentlichen gleicher Temperatur dem zweiten Reaktor zugeführt wird. Das heißt, es erfolgt keine Kompression oder Entspannung des Pre-Restgasstromes, ebenso durchläuft der Pre-Restgasstrom keinen Wärmetauscher zwischen dem ersten Pre-Reaktor und dem zweiten Reaktor. Es muss keine zusätzliche Energie aufgewendet werden, um den Pre-Restgasstrom vor der Zuführung in den zweiten Reaktor zu behandeln. Insbesondere wird der jeweils aus dem ersten Pre-Reaktor und dem zweiten Reaktor gewonnene Gasstrom einer Methanol-Trennanordnung zur Gewinnung und Abzweigung eines Rohmethanolstroms zugeführt. Insbesondere ist jedem Reaktor eine korrespondierende Methanol-Trennanordnung prozesstechnisch nachgeschaltet. Nach dem ersten Pre-Reaktor erfolgt also in einer ersten Methanol-Trennanordnung die Abtrennung eines ersten Rohmethanolstroms und der verbliebende Restgasstrom wird als Pre-Restgasstrom dem zweiten Reaktor zugeführt. Der aus dem zweiten Reaktor gewonnene Gasstrom wird einer zweiten Methanol-Trennanordnung zugeführt und daraus ein zweiter Rohmethanolstrom und der Restgasstrom gewonnen. Insbesondere sind die Methanol-Trennanordnungen Bestandteil einer vorgeschlagenen Anlage zur Synthese von Methanol.

Die vorstehend beschriebene Wasserstoffrückgewinnungsanordnung umfasst insbesondere eine erste Druckwechselabsorptionsvorrichtung (PSA) und eine nachgeschaltete zweite Druckwechselabsorptionsvorrichtung (PSA), wobei aus der ersten PSA ein erster Wasserstoff-Rückführungsstrom und ein PSA-Restgasstrom gewonnen wird. Der aus der ersten PSA gewonnene PSA-Restgasstrom weist insbesondere einen niedrigeren Druck auf als der der ersten PSA zugeführte Restgasstrom. Der PSA-Restgasstrom aus der ersten PSA wird insbesondere mittels eines Kompressors druckerhöht und dann der zweiten PSA zugeführt. Der PSA-Restgasstrom wird insbesondere der zweiten PSA mit min 20 bar und insbesondere mit 30 bar zugeführt. Aus der zweiten PSA wird ein zweiter Wasserstoff-Rückführungsstrom und ein Purgestrom gewonnen. Der Purgestrom weist insbesondere einen niedrigeren molaren Wasserstoffanteil auf als der Restgasstrom. Eine PSA erreicht einen sehr hohen Reinheitsgrad im Wasserstoff-Rückführungsstrom und ist mit einem vergleichsweise geringen Druckabfall verbunden, sodass der Wasserstoff-Rückführungsstrom für die Synthese von eMethanol nur in geringem Maße druckerhöht werden muss. Die erste PSA und die zweite PSA sind insbesondere Bestandteil der vorgeschlagenen Anlage.

Der Vorteil der Aufreinigung des Restgasstrom durch die Wasserstoffrückgewinnungsanordnung mit zwei aufeinanderfolgenden PSA ist, dass besonders viel Wasserstoff aus dem Restgasstrom gewonnen und anschließend der Methanol-Synthese wieder zugeführt werden kann. Der Restgasstrom kann einen hohen Volumenstrom im Vergleich zum Recyclestrom aufweisen (wie vorstehend erläutert), da der Wasserstoff mit hohem Wirkungsgrad aus dem Restgasstrom extrahiert wird. Durch den vergleichsweise geringen Volumenstrom des Recyclestroms kann die dort zum Einsatz kommende Recycle-Kompressoranordnung, welcher sehr energieintensiv im Betrieb ist, besonders klein gehalten werden. Man kann damit auch bewusst die Umsetzung im isothermen zweiten Reaktor verschlechtern, da der Wasserstoff über die zweistufige PSA zu mehr als 99% zurückgeführt werden kann.

Durch die Verwendung von zwei hintereinandergeschalteten PSA beträgt der molare Anteil von Wasserstoff im Wasserstoff-Rückgewinnungsstrom bevorzugt mehr als 90%, insbesondere mehr als 95% und insbesondere mehr als 99%.

Insgesamt kann damit eine Umsetzung von mindestens 95%, bevorzugt mindestens 99%, und insbesondere mindestens 99,5% des eingesetzten Wasserstoffs aus dem H-Strom in eMethanol erzielt werden.

In einer weiteren praktischen Ausführungsform erfolgt eine Druckerhöhung des der Methanol-Reaktoranordnung zugeführten H-Stroms mittels einer Eingangs-Kompressoranordnung erst nach Mischung mit dem CO2-Strom. Mit anderen Worten werden der CO2-Strom und der H-Strom zuerst gemischt und dann in einer Eingangs-Kompressoranordnung verdichtet. Das so gemischte Synthesegas lässt sich leichter auf einen höheren Druck bringen als der H-Strom allein. Die Eingangs-Kompressoranordnung ist insbesondere Teil der vorgeschlagenen Anlage zur Synthese von Methanol.

Insbesondere werden der erste Wasserstoff-Rückführungsstrom und der zweite Wasserstoffrückführungsstrom getrennt der Eingangs-Kompressoranordnung zugeführt. Insbesondere werden der erste Wasserstoff-Rückführungsstrom und der zweite Wasserstoffrückführungsstrom jeweils getrennt einer passenden Druckstufe der Eingangs-Kompressoranordnung zugeführt. Der bereits vorhandene erhöhte Druck der aus der ersten PSA (insbesondere mit 80 bar) und der zweiten PSA (insbesondere mit 30 bar) gewonnenen Wasserstoff-Rückführungsströme kann so genutzt werden und die Kompressorleistung der Eingangs-Kompressoranordnung entsprechend reduziert werden.

Der Wasserstoff-Rückführungsstrom kann alternativ dem H-Strom zugeführt werden, insbesondere stromaufwärts der Eingangs-Kompressoranordnung.

Insbesondere wird der Wasserstoff-Rückführungsstrom dem H-Strom direkt, ohne Durchlauf durch eine zusätzliche Kompressoranordnung, zugeführt und dann zusammen mit dem H-Strom und insbesondere dem CO2-Strom vor Eintritt die Methanol-Reaktoranordnung verdichtet.

Der aus der Wasserstoffrückgewinnungsanordnung gewonnene Purgestrom wird insbesondere einer CO2-Strom-Gewinnungsanlage zugeführt. Bei der CO2-Strom-Gewinnungsanlage handelt es sich insbesondere um die oben beschriebene Anlage zur Gewinnung von CO2 aus einem Rauchgas oder um eine Anlage zur Gewinnung von Bioethanol oder Biomethan. Insgesamt wird so das noch im Verfahren enthaltene und nicht umgesetzte CO2 rückgeführt und zur weiteren Synthese von eMethanol verwendet. Die CO2-Strom-Gewinnungsanlage ist insbesondere Teil der vorgeschlagenen Anlage.

Dabei ist es vorteilhaft, wenn der Druck des Purgestroms nach Verlassen der Wasserstoffrückgewinnungsanordnung mindestens so groß ist wie der Druck an der CO2-Strom-Gewinnungsanlage. Der Druck der zweiten PSA wird insbesondere so gewählt, dass dieser mit dem Druck an der CO2-Strom Gewinnungsanlage übereinstimmt. Insbesondere beträgt der Druck des Purgestroms maximal 20 bar. Etwaige inerte Bestandteile des Purgestroms werden in der CO2-Strom-Gewinnungsanlage herausgefiltert und ausgekreist.

Der aus der Methanol-Reaktoranordnung gewonnene Rohmethanolstrom (welcher auch aus zwei zusammengeführten Rohmethanolströmen aus dem ersten Pre-Reaktor und dem zweiten Pre-Reaktor bestehen kann) wird einer Destillationsanlage zum Gewinnen des Methanols zugeführt. Die Destillationsanlage wird insbesondere mittels Dampf aus der Methanol-Reaktoranordnung, nämlich dem zweiten isothermen Reaktor geheizt. Der dort gewonnene Dampf ist aber nicht ausreichend für die Destillation, deshalb ist hier vorgesehen, dass die Destillationsanlage zur Gewinnung des Methanols zusätzlich elektrisch geheizt wird. Dazu können elektrisch betriebene Reboiler eingesetzt werden. Im Gegensatz zur sonst bekannten Erzeugung von weiterem Dampf zur Schließung der Dampflücke mittels Verbrennung von kohlenstoffhaltigen Material oder der Verbrennung von Wasserstoff entsteht so kein weiteres CO2 bzw. wirkt sich dies nicht ungünstig auf den Wirkungsgrad und die Umsetzung des eingesetzten Wasserstoffs aus. Die Destillationsanlage ist insbesondere Teil der Anlage zur Synthese von Methanol.

Die für die Elektrolyse des H-Stroms aus Wasser verwendete Stromquelle entspricht insbesondere der für die Destillation zur Heizung der Reboiler verwendeten Stromquelle. Insbesondere ist der dafür benötigte Strom CO2-neutral gewonnen.

Ein aus der Destillationsanlage gewonnener Destillations-Purgestrom wird insbesondere einer CO2-Strom-Gewinnungsanlage zugeführt. Insbesondere kann der Destillations-Purgestrom mit dem aus der Wasserstoffgewinnungsanlage gewonnenen Purgestrom zusammengeführt werden und dann der CO2-Strom-Gewinnungsanlage zugeführt werden.

Die Erfindung betrifft auch eine Anlage zur Synthese von eMethanol mit einer Methanol-Reaktoranordnung, welcher ein überwiegend aus Kohlenstoffdioxid bestehender CO2-Strom und ein überwiegend aus Wasserstoff bestehender H-Strom zur Umwandlung in eMethanol zugeführt werden, wobei ein Restgasstrom mit unreagiertem Wasserstoff und unreagiertem Kohlenstoffdioxid aus der Methanol-Reaktoranordnung gewonnen wird. Die Methanol-Reaktoranordnung weist einen ersten, adiabatischen Pre-Reaktor und einen nachgeschalteten zweiten isothermen Reaktor, wobei aus dem ersten Pre-Reaktor ein erster Rohmethanol-Teilstrom gewonnen wird und ein Pre-Restgasstrom, wobei der Pre-Restgasstrom dem zweiten Reaktor zur Gewinnung von Dampf, eines zweiten Rohmethanol-Teilstroms und des Restgasstromes zugeführt wird. Die Anlage weist ferner eine Wasserstoffrückgewinnungsanordnung auf und ein Teil des Restgasstromes wird der Wasserstoff-rückgewinnungsanordnung zugeführt zum Gewinnen eines Wasserstoff-Rückführungsstroms. In Bezug auf weitere Merkmale und Vorteile wird auf die vorstehende Beschreibung verwiesen.

Insbesondere wird der H-Strom aus einer Wasserstoff-Elektrolysevorrichtung gewonnen, wobei die Wasserstoff-Elektrolysevorrichtung Teil der Anlage zur Synthese von eMethanol ist.

Im Folgenden sind in Verbindung mit der Figur eine weitere praktische Ausführungsform und Vorteile beschrieben. Es zeigt:

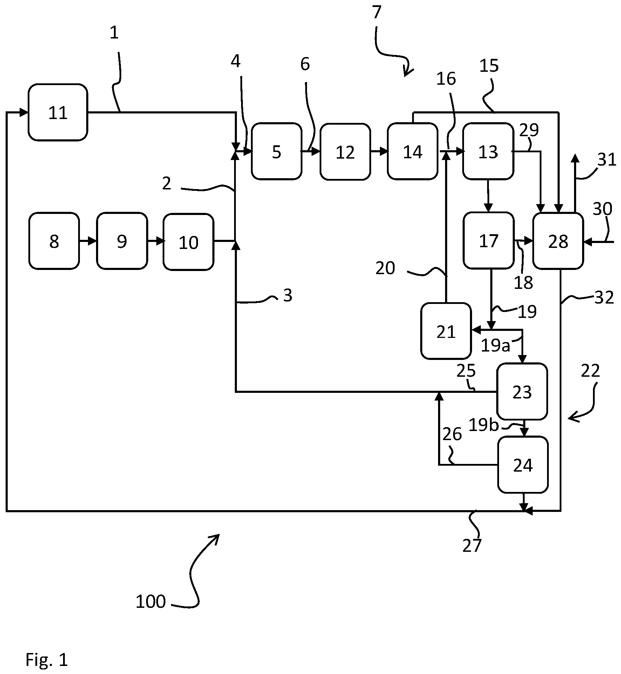

- Fig. 1: schematisch ein Fließbild einer Anlage zur Durchführung eines Verfahrens zur Synthese von Methanol.

Die in der Fig. 1 gezeigte Anlage 100 dient der Synthese von eMethanol und kann gemäß dem vorschlagsgemäßen Verfahren betrieben werden.

Ein im Wesentlichen aus Kohlenstoffdioxid bestehender CO2-Strom 1 und ein im Wesentlichen aus Wasserstoff bestehender H-Strom 2 und ein ebenfalls im Wesentlichen aus Wasserstoff bestehender Wasserstoff-Rückführungsstrom 3 werden zu einem Synthesegasstrom 4 zusammengeführt und durch eine Eingangs-Kompressoranordnung 5 druckerhöht. Der druckerhöhte Synthesegasstrom 6 wird anschließend einer Methanol-Reaktoranordnung 7 zugeführt. Die Eingangs-Kompressoranordnung 5 komprimiert den Synthesegasstrom 4, welcher den H-Strom 2 und den CO2-Strom 1 umfasst, auf 75 bar bis 100 bar, vorzugsweise 80 bar.

Der H-Strom 2 wird in einer Wasserstoff-Elektrolysevorrichtung 8 gefolgt von einer Entwässerungseinrichtung 9 und einer Sauerstoffentfernungseinrichtung 10 gewonnen.

Der CO2-Strom 1 wird in einer CO2-Strom-Gewinnungsanlage 11 gewonnen. Es handelt sich bei der CO2-Strom-Gewinnungsanlage 11 um eine Anlage zur Entfernung von CO2 aus einem Rauchgas oder um eine Anlage zur Gewinnung von Bioethanol oder Biomethan.

Die Methanol-Reaktoranordnung 7 umfasst einen ersten adiabatischen Pre-Reaktor 12 und einen seriell dazu angeordneten zweiten isothermen Reaktor 13.

Der druckerhöhte Synthesegasstrom 6 wird dem ersten adiabatischen Pre-Reaktor 12 zur Gewinnung von eMethanol zugeführt und dort wird bereits ein Teil des CO2 zusammen mit dem Wasserstoff in Rohmethanol umgewandelt. Um das Rohmethanol zu extrahieren wird das Gasgemisch aus dem ersten Pre-Reaktor 12 einer ersten Methanol-Trennvorrichtung 14 zur Gewinnung eines ersten Rohmethanol-Teilstroms 15, im Wesentlichen bestehend aus Rohmethanol, und eines Pre-Restgasstromes 16 mit unreagierten Restgasen zugeführt.

Der Pre-Restgasstrom 16 wird direkt dem zweiten isothermen Reaktor 13 zur weiteren Gewinnung von eMethanol zugeführt. Der aus dem zweiten Reaktor 13 gewonnene Gasstrom wird einer zweiten Methanol-Trennvorrichtung 17 zugeführt zur Gewinnung eines zweiten Rohmethanol-Teilstroms 18, im Wesentlichen bestehend aus Rohmethanol, und eines Restgasstromes 19 mit unreagierten Restgasen, wie unreagiertem Wasserstoff und unreagiertem CO2.

Ein Teil des Restgasstromes 19 wird als Recyclestrom 20 der Methanol-Reaktoranordnung 7 wieder zugeführt. Der Recyclestrom 20 wird dazu einer Recycle-Kompressoranordnung 21 zugeführt, zur Druckerhöhung des Recyclestroms 20. Der Recyclestrom 20 wird zusammen mit dem Pre-Restgasstrom 16 direkt dem zweiten Reaktor 13 zur weiteren Umsetzung des unreagierten CO2 und H2 zugeführt.

Ein verbleibende Teil des Restgasstromes 19a - welcher nach Abzweigung des Recyclestroms 20 verbleibt - wird einer Wasserstoffrückgewinnungsanordnung 22 zugeführt. Die Wasserstoffrückgewinnungsanordnung 22 umfasst eine erste PSA 23 und eine zweite PSA 24, wobei die erste PSA 23 und die zweite PSA 24 hintereinandergeschaltet sind. Aus der ersten PSA 23 wird ein erster Wasserstoff-Rückführungsstrom 25, welcher im Wesentlichen aus Wasserstoff besteht, und aus der zweiten PSA 24 wird ein zweiter Wasserstoff-Rückführungsstrom 26 gewonnen, welcher im Wesentlichen aus Wasserstoff besteht. Der erste Wasserstoff-Rückführungsstrom 25 und zweite Wasserstoff-Rückführungsstrom 26 werden zum Wasserstoff-Rückführungsstrom 3 zusammengeführt und dann dem H-Strom 2 stromaufwärts der Eingangs-Kompressoranordnung 5 zugeführt. Alternativ zu der hier gezeigten Ausführungsform können der erste Wasserstoff-Rückführungsstrom 25 und der zweite Wasserstoff-Rückführungsstrom 26 getrennt einer passenden Druckstufe der Eingangs-Kompressoranordnung zugeführt werden.

Ein aus der ersten PSA gewonnener PSA-Restgasstrom 19b weist einen niedrigeren Druck auf als der verbleibende Restgasstrom 19a, welcher der ersten PSA 23 zugeführt wird. Der PSA-Restgasstrom 19b wird nach einer Druckerhöhung dann der zweiten PSA 24 zugeführt.

Aus der Wasserstoff-Rückgewinnungsanordnung 22 wird ein an Wasserstoff abgereicherter Purgestrom 27 gewonnen. Dieser Purgestrom 27 enthält im Wesentlichen unreagiertes CO2 und inerte Bestandteile.

Ohne weitere Kompression wird der Purgestrom 27 der CO2-Strom-Gewinnungsanlage 11 zugeführt. Das in der CO2-Gewinnungsanlage gewonnene CO2 wird gefiltert und gereinigt, wobei auch die inerten Bestandteile zum Teil ausgekreist werden (nicht dargestellt).

Die Rohmethanol-Teilströme 15, 18 werden zur Gewinnung von eMethanol einer Destillationsanlage 28 zugeführt. In der Destillationsanlage 28 erfolgt ein Erhitzen zur Entfernung von Wasser aus dem Rohmethanol. Die dafür erforderliche Wärmemenge wird durch Dampf 29 zur Verfügung gestellt, welcher aus der exothermen Reaktion im zweiten isothermen Reaktor 13 gewonnen wird. Um die Dampflücke zu schließen, erfolgt die Destillation zusätzlich unter Einsatz elektrischer Energie 30 mit elektrisch betriebenen Reboilern.

Das aus der Destillationsanlage 28 gewonnene eMethanol 31 wird zur weiteren Verwendung abgeführt. Ferner wird ein aus der Destillationsanlage 28 gewonnener Destillations-Purgestrom 32 dem Purgestrom 27 zugeführt und zusammen hiermit der CO2-Strom-Gewinnungsanlage 11 zugeführt.

### Bezugszeichenliste

- 100: Anlage

- 1: CO2-Strom
- 2: H-Strom
- 3: Wasserstoff-Rückführungsstrom
- 4: Synthesegasstrom
- 5: Eingangs-Kompressoranordnung
- 6: druckerhöhter Synthesegasstrom
- 7: Methanol-Reaktoranordnung
- 8: Wasserstoff-Elektrolysevorrichtung
- 9: Entwässerungseinrichtung
- 10: Sauerstoffentfernungseinrichtung
- 11: CO2-Strom-Gewinnungsanlage
- 12: erster Pre-Reaktor
- 13: zweiter Reaktor
- 14: erster Methanol-Trennvorrichtung
- 15: erster Rohmethanol-Teilstrom
- 16: Pre-Restgasstrom
- 17: zweite Methanol-Trennvorrichtung
- 18: zweiter Rohmethanol-Teilstrom
- 19: Restgasstrom
- 19a: verbleibender Restgasstrom
- 19b: PSA-Restgasstrom
- 20: Recyclestrom
- 21: Recycle-Kompressoranordnung
- 22: Wasserstoff-Rückgewinnungsanordnung
- 23: erste PSA
- 24: zweite PSA
- 25: erster Wasserstoff-Rückführungsstrom
- 26: zweiter Wasserstoff-Rückführungsstrom
- 27: Purgestrom
- 28: Destillationsanlage
- 29: Dampf
- 30: elektrische Energie
- 31: Methanol
- 32: Destillations-Purgestrom

## Patentansprüche

1. Verfahren zur Synthese von eMethanol (31), wobei ein überwiegend aus Kohlenstoffdioxid bestehender CO2-Strom (1) und ein überwiegend aus Wasserstoff bestehender H-Strom (2) einer Methanol-Reaktoranordnung (7) zur Umwandlung in eMethanol (31) zugeführt werden, wobei ein Restgasstrom (19) mit unreagiertem Wasserstoff und unreagiertem Kohlenstoffdioxid aus der Methanol-Reaktoranordnung (7) hervorgeht, **dadurch gekennzeichnet, dass**
die Methanol-Reaktoranordnung (7) einen ersten adiabatischen Pre-Reaktor (12) und einen nachgeschalteten zweiten isothermen Reaktor (13) aufweist, wobei aus dem ersten Pre-Reaktor (12) ein erster Rohmethanol-Teilstrom (15) gewonnen wird und ein Pre-Restgasstrom (16), wobei der Pre-Restgasstrom (16) dem zweiten Reaktor (13) zur Gewinnung von Dampf (29), eines zweiten Rohmethanol-Teilstroms (18) und des Restgasstromes (19) zugeführt wird und wobei ein Teil des Restgasstromes (19a) einer Wasserstoffrückgewinnungsanordnung (22) zugeführt wird zum Gewinnen eines Wasserstoff-Rückführungsstroms (3).

2. Verfahren nach dem vorstehenden Anspruch,
**dadurch gekennzeichnet, dass**
ein Teil des Restgasstromes (19) als Recyclestrom (20) zur Methanol-Reaktoranordnung (7) rückgeführt wird.

3. Verfahren nach dem vorstehenden Anspruch,
**dadurch gekennzeichnet, dass**
der Recyclestrom (20) der Methanol-Reaktoranordnung (7) stromabwärts des ersten Pre-Reaktors (12) und stromaufwärts des zweiten Reaktors (13) zugeführt wird.

4. Verfahren nach einem der vorstehenden Ansprüche,
**dadurch gekennzeichnet, dass**
der Recyclefaktor des zweiten Reaktors (13) maximal 3,5 beträgt.

5. Verfahren nach einem der vorstehenden Ansprüche,
**dadurch gekennzeichnet, dass**
der Pre-Restgasstrom (16) direkt dem zweiten Reaktor (13) zugeführt wird.

6. Verfahren nach einem der vorstehenden Ansprüche,
**dadurch gekennzeichnet, dass**
die Wasserstoffrückgewinnungsanordnung (22) eine erste Druckwechselabsorptionsvorrichtung (23) und eine nachgeschaltete zweite Druckwechselabsorptionsvorrichtung (24) umfasst, wobei aus der ersten Druckwechselabsorptionsvorrichtung (23) ein erster Wasserstoff-Rückführungsstrom (25) gewonnen wird und aus der zweiten Druckwechselabsorptionsvorrichtung (24) ein zweiter Wasserstoff-Rückführungsstrom (26) gewonnen wird.

7. Verfahren nach dem vorstehenden Anspruch,
**dadurch gekennzeichnet, dass**
ein aus der ersten Druckwechselabsorptionsvorrichtung (23) gewonnener Restgaststrom (19b) vor Eingang in die zweite Druckwechselabsorptionsvorrichtung (24) druckerhöht wird.

8. Verfahren nach einem der vorstehenden Ansprüche,
**dadurch gekennzeichnet, dass**
der molare Anteil von Wasserstoff im Wasserstoff-Rückführungsstrom (3) größer als 99% ist.

9. Verfahren nach einem der vorstehenden Ansprüche,
**dadurch gekennzeichnet, dass**
eine Druckerhöhung des der Methanol-Reaktoranordnung (7) zugeführten H-Stroms (2) durch eine Eingangs-Kompressoranordnung (5) erst nach Mischung mit dem CO2-Strom (1) erfolgt.

10. Verfahren nach einem der vorstehenden Ansprüche 6 bis 8,
**dadurch gekennzeichnet, dass**
der erste Wasserstoffrückführungsstrom (25) und der zweite Wasserstoff-rückführungsstrom (26) separat jeweils einer passenden Druckstufe der Eingangs-Kompressoranordnung (5) zugeführt werden.

11. Verfahren nach einem der vorstehenden Ansprüche,
**dadurch gekennzeichnet, dass**
aus der Wasserstoffrückgewinnungsanordnung (22) ein Purgestrom (27) gewonnen wird, welcher einer CO2-Strom-Gewinnungsanlage (11) zugeführt wird.

12. Verfahren nach dem vorstehenden Anspruch,
**dadurch gekennzeichnet, dass**
der Druck des Purgestroms (27) nach Verlassen der Wasserstoffrückgewinnungsanordnung (22) mindestens so groß ist wie der Druck an der CO2-Strom-Gewinnungsanlage (11).

13. Verfahren nach einem der vorstehenden Ansprüche,
**dadurch gekennzeichnet, dass**
die Rohmethanol-Teilströme (15, 18) aus dem ersten Pre-Reaktor (12) und dem zweiten Reaktor (13) einer Destillationsanlage (28) zum Gewinnen des eMethanol (31) zugeführt werden, wobei die Destillationsanlage (28) mittels Dampf (29) aus der Methanol-Reaktoranordnung (7) geheizt wird und zusätzlich elektrisch geheizt wird.

14. Verfahren nach dem vorstehenden Anspruch,
**dadurch gekennzeichnet, dass**
ein aus der Destillationsanlage (28) gewonnener Destillations-Purgestrom (32) einer CO2-Strom-Gewinnungsanlage (11) zugeführt wird.

15. Anlage zur Synthese von eMethanol (31) mit einer Methanol-Reaktoranordnung (7), welcher ein überwiegend aus Kohlenstoffdioxid bestehender CO2-Strom (1) und ein überwiegend aus Wasserstoff bestehender H-Strom (2) zur Umwandlung in eMethanol (31) zugeführt werden, wobei ein Restgasstrom (19) mit unreagiertem Wasserstoff und unreagiertem Kohlenstoffdioxid aus der Methanol-Reaktoranordnung (7) gewonnen wird,
**dadurch gekennzeichnet, dass**
die Methanol-Reaktoranordnung (7) einen ersten, adiabatischen Pre-Reaktor (12) und einen nachgeschalteten zweiten isothermen Reaktor (13) aufweist, wobei aus dem ersten Pre-Reaktor (12) ein erster Rohmethanol-Teilstrom (15) gewonnen wird und ein Pre-Restgasstrom (16), wobei der Pre-Restgasstrom (16) dem zweiten Reaktor (13) zur Gewinnung von Dampf (29), eines zweiten Rohmethanol-Teilstroms (18) und des Restgasstromes (19) zugeführt wird und wobei die Anlage (100) eine Wasserstoffrückgewinnungsanordnung (22) umfasst und ein Teil des Restgasstromes (19a) der Wasserstoffrückgewinnungsanordnung (22) zugeführt wird zum Gewinnen eines Wasserstoff-Rückführungsstroms (3).
